Europäisches Patentamt

European Patent Office

Office européen des brevets

(19)

(11) Publication number: **0 391 214**

**A1**

(12) **EUROPEAN PATENT APPLICATION**

(21) Application number: **90105811.5**

(51) Int. Cl.5: **C07K 15/12, A61K 37/02**

(22) Date of filing: **27.03.90**

| | |
|---|---|
| The microorganism(s) has (have) been deposited with American Type Culture Collection under number(s) CCL 92. | (71) Applicant: **CENTRE NATIONAL DE LA RECHERCHE SCIENTIFIQUE**<br>**15, quai Anatole France**<br>**F-75007 Paris(FR)** |
| (30) Priority: **03.04.89 US 332688**<br>**24.07.89 US 384351** | (72) Inventor: **Harel, Louise**<br>**15 Rue Thilboumery** |
| (43) Date of publication of application:<br>**10.10.90 Bulletin 90/41** | **Paris 75015(FR)**<br>Inventor: **Blat, Christiane**<br>**68 Avenue du Chateau** |
| (84) Designated Contracting States:<br>**AT BE CH DE DK ES FR GB GR IT LI LU NL SE** | **Bourg La Reine, 92340(FR)**<br>Inventor: **Chatelain, Gilles**<br>**42 Avenue Jean Pennin**<br>**Sceaux 92330(FR)**<br>Inventor: **Bohlen, Peter**<br>**2 Amalfi Drive**<br>**Cortlandt, New York 10566(US)** |
| | (74) Representative: **Wächtershäuser, Günter, Dr.**<br>**Tal 29**<br>**D-8000 München 2(DE)** |

(54) Bifunctional protein designated IDF 45.

(57) A novel bifunctional protein, designated IDF$_{45}$, is disclosed. IDF$_{45}$ is isolated from mouse 3T3 cells by a sequence of purification steps. In one aspect of the invention, IDF$_{45}$ functions as a cellular growth inhibitor. The cellular growth inhibitor may be useful in the inhibition of growth in fibroblasts, endothelial cells and other cells, as well as in the inhibition of DNA synthesis in those cells. In another aspect of the invention, IDF$_{45}$ functions as a binding protein for insulin-like growth factor I. As such, IDF$_{45}$ may be useful to inhibit the various effects of insulin-like growth factor I.

EP 0 391 214 A1

# BIFUNCTIONAL PROTEIN DESIGNATED IDF$_{45}$

## SUMMARY OF THE INVENTION

This application is a continuation-in-part of copending application serial number 07/332,688, filed on April 3, 1989.

This invention relates to a novel bifunctional protein, named inhibitory diffusible factor ("IDF$_{45}$"), which has been purified to homogeneity from medium conditioned by dense cultures of mouse 3T3 cells. Sodium dodecyl sulfate-polyacrylamide gel electrophoresis ("SDS-PAGE") of bioactive material purified by reverse-phase high performance liquid chromatography ("HPLC") shows a single band corresponding to a protein with a molecular weight of 45 kD. The molecule is a monomer. The pl of the molecule, as determined by isoelectric focusing, is about 6.5. The amino-terminal sequence of the protein is shown to be Ser-Ala-Gly-Ala-Val-Gly-Ala-Gly-Pro-Val-Val-Arg.

IDF$_{45}$ is a growth inhibitory protein which inhibits DNA synthesis in chicken embryo fibroblasts in a dose-dependent manner (ED$_{50}$: 40 ng/ml, approximately 1 nM). The protein also inhibits growth of cells such as fibroblasts and endothelial cells. In addition, IDF$_{45}$ functions as a binding protein for insulin-like growth factor I ("IGF-I"). As such, IDF$_{45}$ inhibits the various effects of IGF-I.

The chemical properties of IDF$_{45}$, including molecular weight and amino-terminal sequence, clearly distinguish it from other known growth inhibitory and IGF-I binding proteins.

## BACKGROUND OF THE INVENTION

The growth rate of mouse 3T3 cells in vitro decreases as the cell density of the culture increases. Some evidence (References 1,2) suggests that this density-dependent inhibition of growth is the result of the secretion of inhibitory molecule(s) from cells in dense cultures. A number of proteins have recently been characterized which possess potent growth inhibitory properties towards a variety of cell types in culture and which may play a role in the regulation of density-dependent growth of cells in culture.

Among these proteins are the family of type-beta transforming growth factors ("TGF") (3,4,5,6), the interferons (7), tumor necrosis factors ("TNF") (8,9,10,11) and interleukin-1 (12).

Furthermore, from medium conditioned by dense cultures of 3T3 cells, a 13 kD inhibitory molecule designated fibroblast growth regulator ("FGR") was recently purified to apparent homogeneity (13) and appears to be structurally related to a mammary-derived growth inhibitor (14,15,16).

Another growth inhibitory protein secreted from dense 3T3 cells was discovered and originally thought to have a molecular weight of approximately 40 kD (17). The protein was then partially purified by reverse-phase FPLC, determined by SDS-PAGE to have a molecular weight of 45 kD and was named IDF$_{45}$ (18). It was shown that this partially purified IDF$_{45}$ acted in the GI phase of the cell cycle and inhibited the early stimulation of RNA synthesis induced by growth factor (18). While IDF$_{45}$ was found to inhibit reversibly DNA synthesis and growth in chick embryo fibroblasts ("CEF"), little growth inhibitory activity was observed in CEF tranformed by v-src oncogene (19).

Based on this prior work, it is an object of this invention to purify IDF$_{45}$ to homogeneity and to establish its amino-terminal sequence. It is a further object of this invention to establish the biological activity of IDF$_{45}$.

Another aspect of this invention is related to the effect of IDF$_{45}$ on IGF-I. IGF-I is a peptide referred to before 1987 as somatomedin A or C (20). IGF-I, together with IGF-II, represent a group of peptides having the ability to: 1) stimulate proteoglycan synthesis in cartilage (by incorporation of sulfate into cartilage); 2) mimic the action of insulin in adipose tissue and in the diaphragm without being affected by anti-insulin serum; and 3) promote cell division (by stimulating the incorporation of thymidine into human skin fibroblasts).

Several different proteins which bind to IGF (I and/or II) are known to be present in serum (21,22,23). They form large complexes (of about 150 kD) which are under growth hormone control, as well as smaller complexes (of about 40 kD). After SDS-PAGE, different molecular forms have been observed from 41.5 kD to 24 kD (24). IGF binding proteins of 41.5 to 24 kD are also known to be secreted by cells in culture (25,26,27,28,29).

Some of these IGF binding proteins have been recently purified and characterized (25,26,30,31). The amino-terminal sequences of 30-34 kD IGF binding proteins of BRL 3A rat cell line (26) and of human amniotic fluid (30,31) are different from the $IDF_{45}$ amino-terminal sequence set forth above.

Accordingly, it is another object of this invention to ascertain whether $IDF_{45}$ is an IGF-I binding protein and, if so, to determine the extent of any inhibition by $IDF_{45}$ of IGF-I. It is a still further object of this invention to determine any interrelationship between the inhibitory effect of $IDF_{45}$ on serum and on IGF-I.

BRIEF DESCRIPTION OF THE DRAWINGS

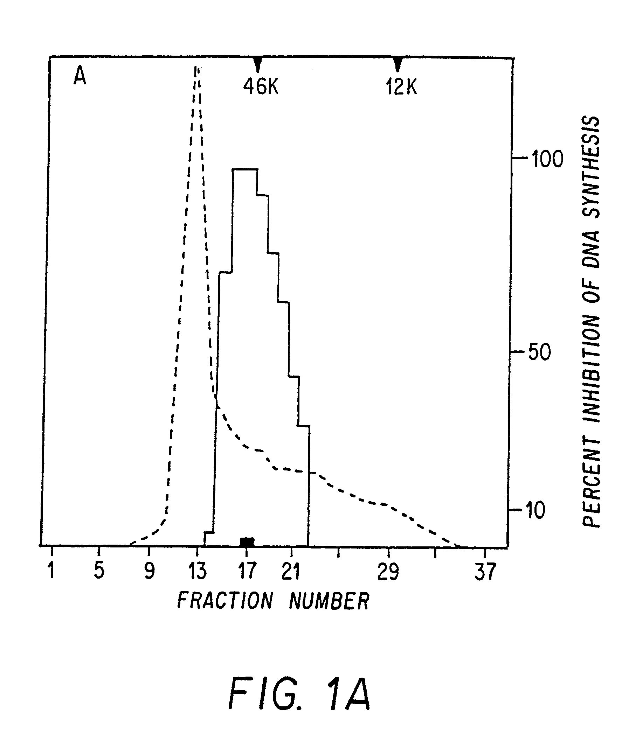

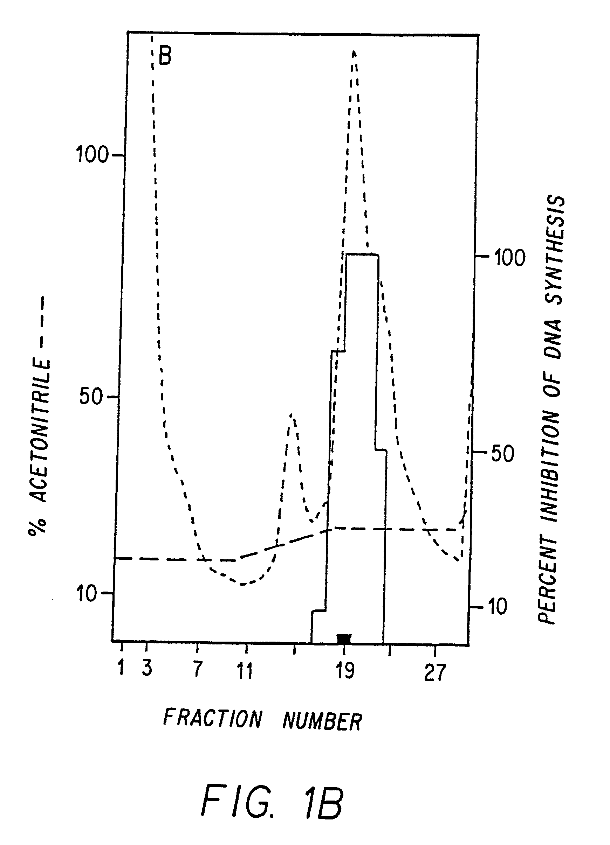

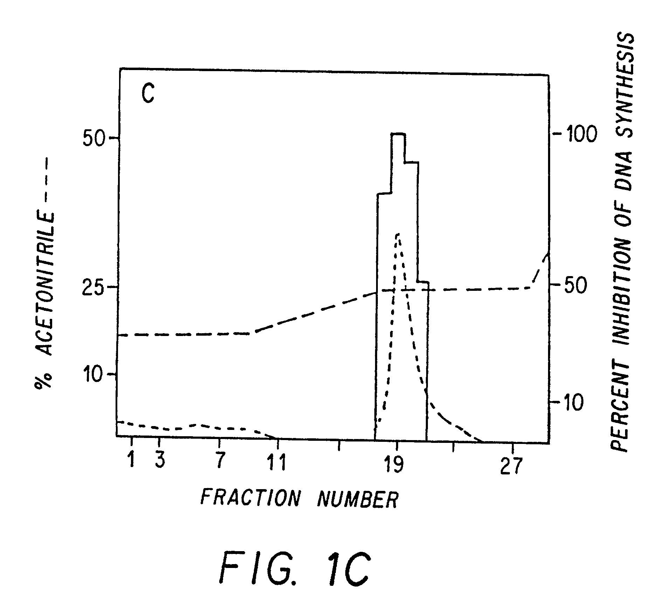

Figure 1 depicts the purification of $IDF_{45}$ in three stages:

A/ Macromolecules of the medium (11.6 mg) solubilized in 1M acetic acid are chromatographed on a P150 Bio-Gel column (20x5 cm), and eluted with 1M acetic acid at a flow rate of 21 ml/hour. Each fraction (8.5 ml) is lyophilized and an aliquot is tested for inhibitory activity of DNA synthesis. Percent inhibition of DNA synthesis is indicated by (—), while the protein eluted from the column (as indicated by absorbance at 280nm) is indicated by (--).

B/ The inhibitory fraction 17 (500 μg protein) from the Bio-Gel P150 chromatography shown in Fig. 1A is solubilized in 1 ml of 0.05% trifluoroacetic acid ("TFA") , adjusted to 17% acetonitrile in TFA and fractionated by reverse-phase HPLC on a $C_8$ Pro RPC HR 5/2 column. Elution is achieved with a multilinear gradient of acetonitrile in 0.05% TFA (17-24% acetonitrile for 9 minutes, 24% acetonitrile for 10 minutes, and 24-100% acetonitrile for 15 minutes). Each fraction (0.3 ml) is lyophilized and an aliquot is tested for inhibitory activity on DNA synthesis. Percent inhibition on DNA synthesis is indicated by (—), while the percent of acetonitrile is indicated by (--) and the protein eluted from the column (280 nm) is indicated by (----).

C/ The fraction 19 (about 5 μg protein) from the reverse-phase FPLC shown in Figure 1B is adjusted to 17% acetonitrile in 0.05% TFA and immediately rechromatographed in the same FPLC system. Elution is achieved with the same gradient. Fractions (0.3 ml) are lyophilized and an aliquot of each is tested for inhibitory activity on DNA synthesis. Percent inhibition on DNA synthesis is indicated by (—), while the percent of acetonitrile is indicated by (--) and the protein eluted from the column (280 nm) is indicated by (----).

Figure 2 depicts the effect of $IDF_{45}$ on bFGF-stimulated endothelial cell growth. Data points are means of duplicate determinations. Individual points do not differ from mean by more than 5%. Cell density in the absence of added bFGF and $IDF_{45}$ is approximately 23,000 cells/well.

Figure 3 depicts the molecular weight analysis on SDS-PAGE of $IDF_{45}$ purified by the second reverse phase FPLC as follows:

Lane 1: $IDF_{45}$; Lane 2: Protein standards -phosphorylase b, 94 kD; bovine serum albumin, 67 kD; ovalbumin, 46 kD; carbonic anhydrase, 30 kD; soybean trypsin inhibitor, 20 kD; lactalbumin, 14 kD.

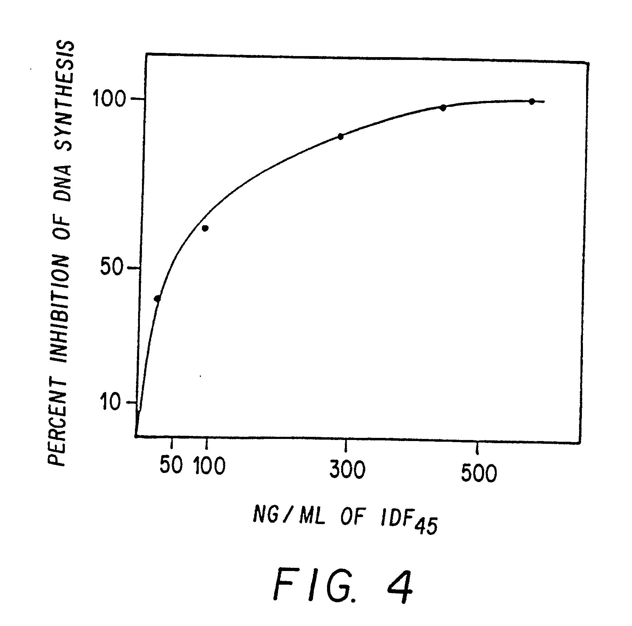

Figure 4 depicts the inhibitory activity of $IDF_{45}$ on CEF in the form of a dose response curve. The inhibitory activity is determined by the percent inhibition of the increase of $^{14}C$-thymidine incorporation into CEF caused by 1% serum. In the absence of serum, $^{14}C$-thymidine incorporation is between 15 and 25% of the incorporation in the presence of serum. DNA synthesis is 100% inhibited in test cells when the readings for $^{14}C$-thymidine incorporated into cells are the same for control cells not stimulated by serum and for growth-stimulated test cells inhibited with $IDF_{45}$.

Figure 5 depicts the inhibition of DNA synthesis in 3T3 cells by $IDF_{45}$ in the presence or absence of monoclonal antibody 2A4. Inhibition is determined by monitoring the extent of the incorporation of $^{14}C$-thymidine into DNA in the test cells.

Figure 6 depicts the stimulation of growth of quiescent CEF upon the addition of IGF-I or serum in the absence of $IDF_{45}$. The stimulation of growth is indicated by the increase of $^{14}C$-thymidine incorporation into CEF caused by IGF-I (-Δ-) or serum (-▲-).

Figures 7 and 8 depict the inhibition by $IDF_{45}$ of IGF-I stimulation. In Figure 7, IGF-I (30ng/ml) is added to quiescent CEF in the absence or presence of different concentrations of purified $IDF_{45}$. The inhibitory activity is determined by the percent inhibition of the increase by IGF-I of $^{14}C$-thymidine incorporation in the cells. DNA synthesis is 100% inhibited in the presence of $IDF_{45}$ when the readings for $^{14}C$-thymidine incorporated into cells are the same.

In Figure 8, either no $IDF_{45}$ (-●-), 1 U (-▲-), or 1.5 U (-Δ-) of $IDF_{45}$ are added to quiescent CEF with different concentrations of IGF-I. The inhibitory activity is determined according to the method of Figure 7.

Figure 9 depicts the detection of binding of $IDF_{45}$ to IGF-I as follows:

A/ $IDF_{45}$ is partially purified by reverse-phase FPLC, as described in the description of Figure 1A and B. Proteins of the most inhibitory fractions obtained after the first reverse-phase FPLC are adjusted to 17% acetonitrile in 0.05% TFA and injected into a reverse-phase column (C8 Pro RPC HR5/2 Pharmacia column). Elution is achieved with a multilinear gradient of acetonitrile in 0.05% TFA (17-24% acetonitrile for 9 minutes, 24% acetonitrile for 10 minutes, 24-100% acetonitrile for 15 minutes). Each fraction (0.3 ml) is lyophilized and an aliquot is tested for inhibitory activity on DNA synthesis. Percent inhibition on DNA synthesis is indicated by (—), while the percent of acetonitrile is indicated by (--) and the protein eluted from the column (280 nm) is indicated by (----).

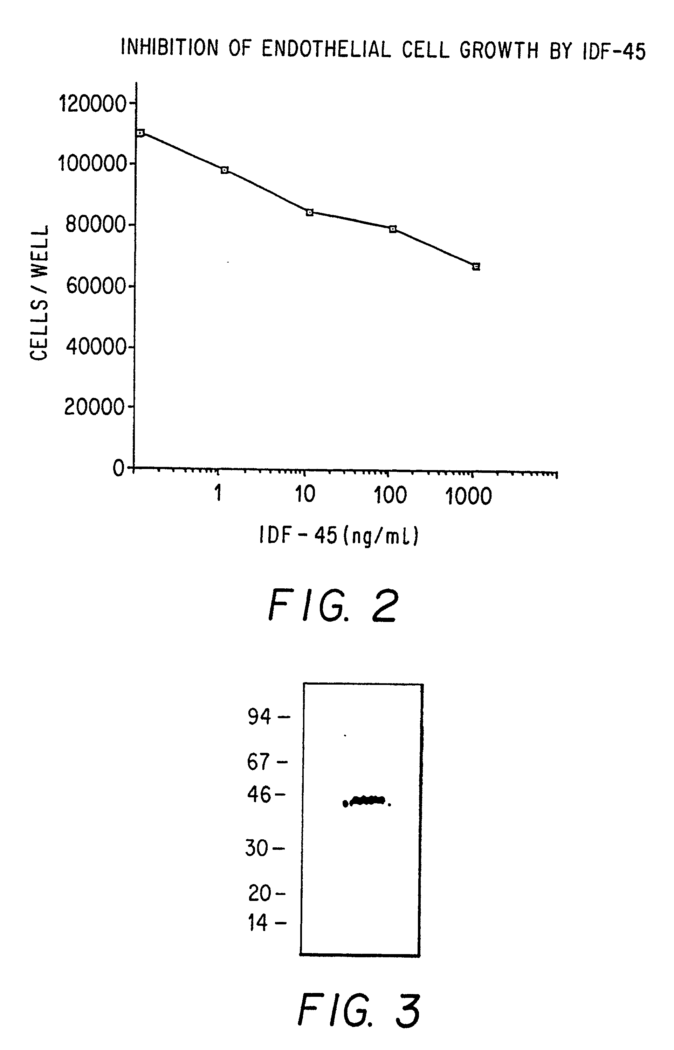

B/ SDS-PAGE of IDF$_{45}$: About 50 ng of protein from fraction 19 of the second reverse-phase FPLC are separated on PhastGel gradient 10-15 and revealed by silver staining. The same protein standards are used as for Figure 3.

C/ Detection of IFG-I binding activity by IDF$_{45}$: About 250 ng of IDF$_{45}$ are submitted to SDS-PAGE, Western-blotted and processed as described by Hossenlopp et al. (32). Binding of the protein to IGF-I is detected by autoradiography after incubation of the protein with $^{125}$I-IGF-I.

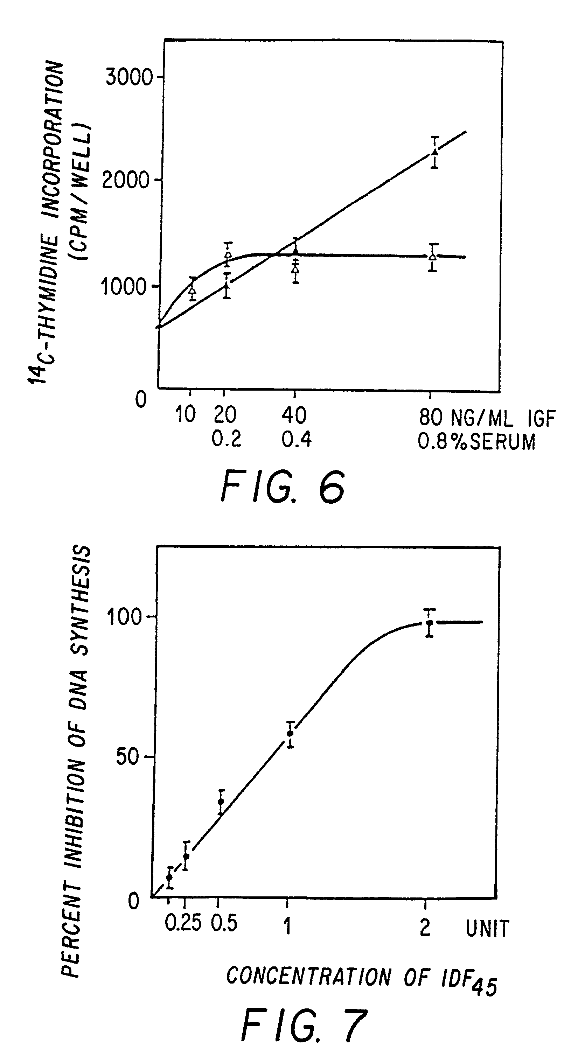

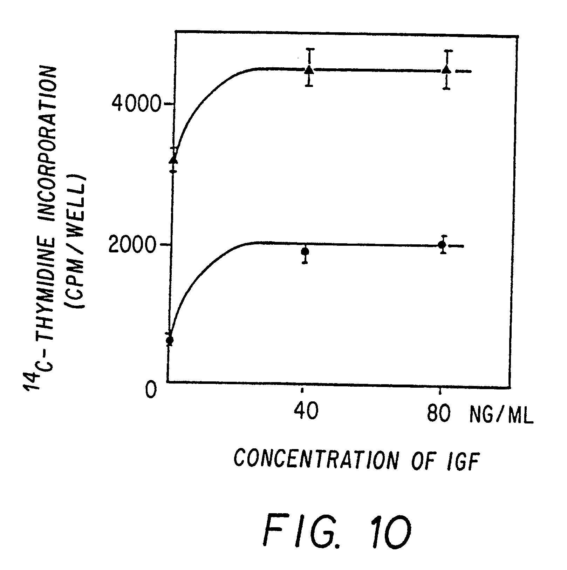

Figure 10 depicts the additive effect of serum and IGF-I on DNA synthesis in the absence of IDF$_{45}$. DNA synthesis in the presence of IGF-I alone (-●-) or IGF-I plus serum (-▲-) is determined according to the method of Figure 7.

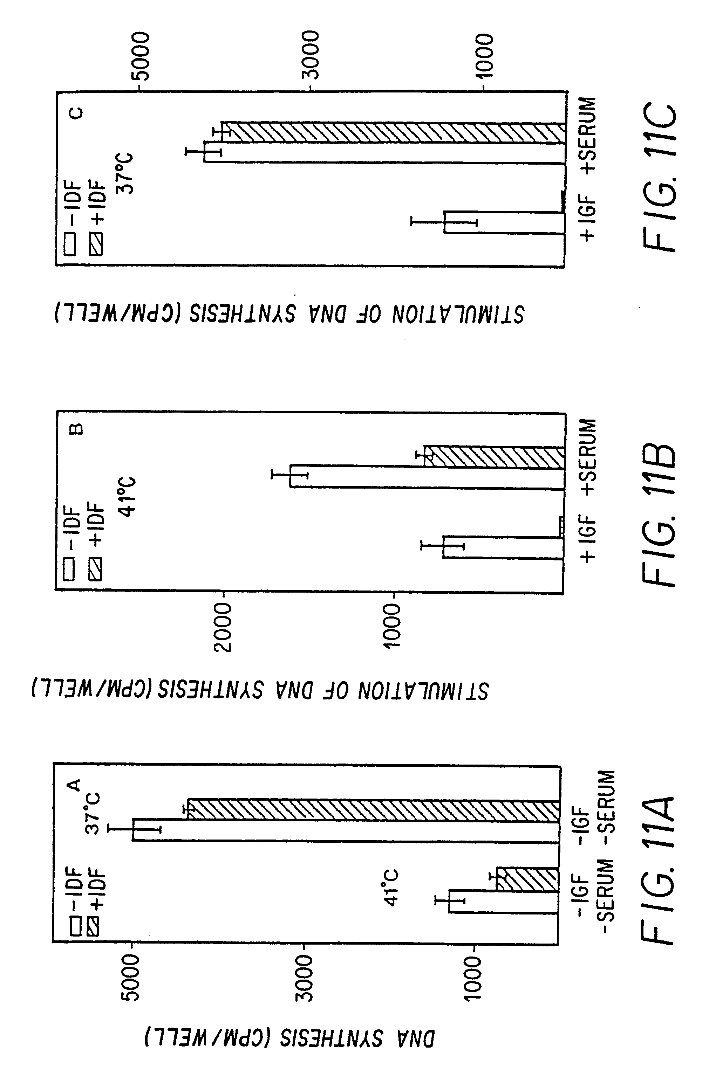

Figure 11 depicts the effect of IDF$_{45}$ on stimulation induced by serum and IGF-I in transformed CEF as follows:

A/ NY68 infected CEF cells are maintained in serum free medium at 41°C or transferred to 37°C; 4 hours later, 1.5 U IDF$_{45}$ are added and cells labelled with $^{14}$C-thymidine between 5 and 24 hours after addition of IDF$_{45}$. DNA synthesis in cells with and without IDF$_{45}$ is measured in cpm/well.

B/ NY68 infected CEF cells are maintained at 41°C and stimulated by IGF-I (30ng/ml) or serum (1%) in the presence or absence of 1.5 U of IDF$_{45}$. Cells are labelled with $^{14}$C-thymidine between 5 and 24 hours after addition of growth factors and IDF$_{45}$. The inhibitory activity is measured according to the method of Figure 7.

C/ NY68 infected CEF cells are transferred from 41°C to 37°C, and 4 hours later, they are stimulated by IGF-I (30ng/ml) or serum (1%) in the presence or absence of 1.5 U of IDF$_{45}$. Cells are labelled with $^{14}$C-thymidine between 5 and 24 hours after addition of growth factors and IDF$_{45}$. The inhibitory activity is measured according to the method of Figure 7.

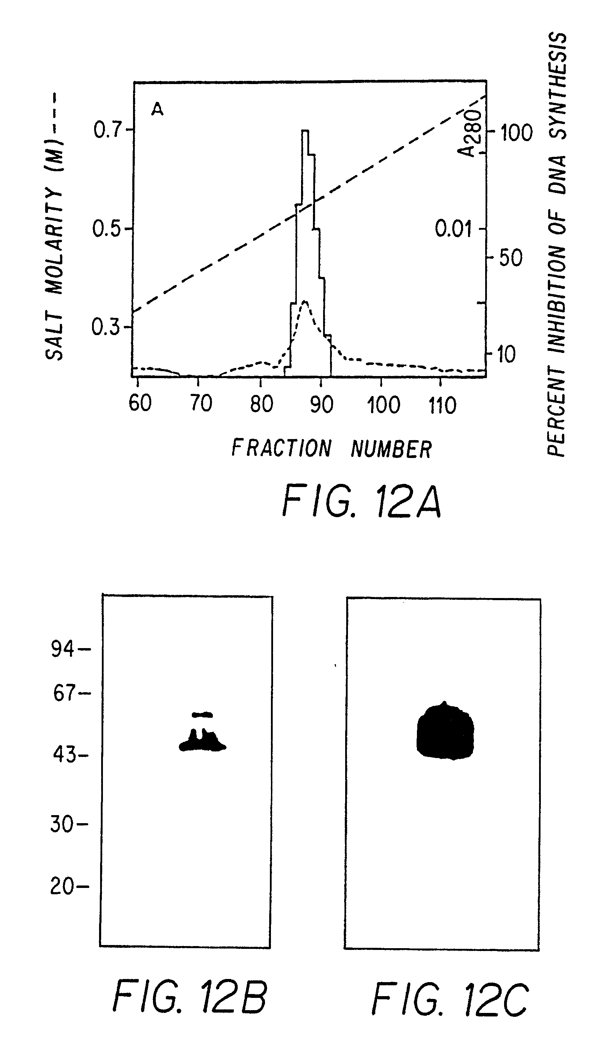

Figure 12 depicts the purification of IDF$_{45}$ by cation exchange FPLC.

A/ IDF$_{45}$ is partially purified as previously described in the description of Figure 1A and B. Proteins of the most inhibitory fractions obtained after the first reverse-phase FPLC are then subjected to cation exchange FPLC by treatment of the proteins with $CH_3COONH_4$ (10mM) and $CH_3COOH$ (50mM), followed by immediate injection into a Mono S HR 5/5 column (Pharmacia) equilibrated with acetonitrile (24%) $CH_3COONH_4$ (10mM) and $CH_3COOH$ (50mM). The elution is achieved with a gradient of $CH_3COONH_4$ - (10mM-1M in 70 minutes) in acetonitrile 24%, $CH_3COONH_4$ (50mM) (gradient indicated by (--)).

The flow rate is 0.6 ml/minute. Each lyophilized fraction (0.3 ml) is tested for its inhibitory activity toward DNA synthesis stimulated by 1% serum. Percent inhibition on DNA synthesis is indicated by (—) and the protein eluted from the column (280nm) is indicated by (----).

B/ SDS-PAGE of IDF$_{45}$: About 50ng of proteins from fraction 87 of the cation exchange FPLC are separated on PhastGel gradient 10-15 and silver stained. The same protein standards are used as for Figure 3.

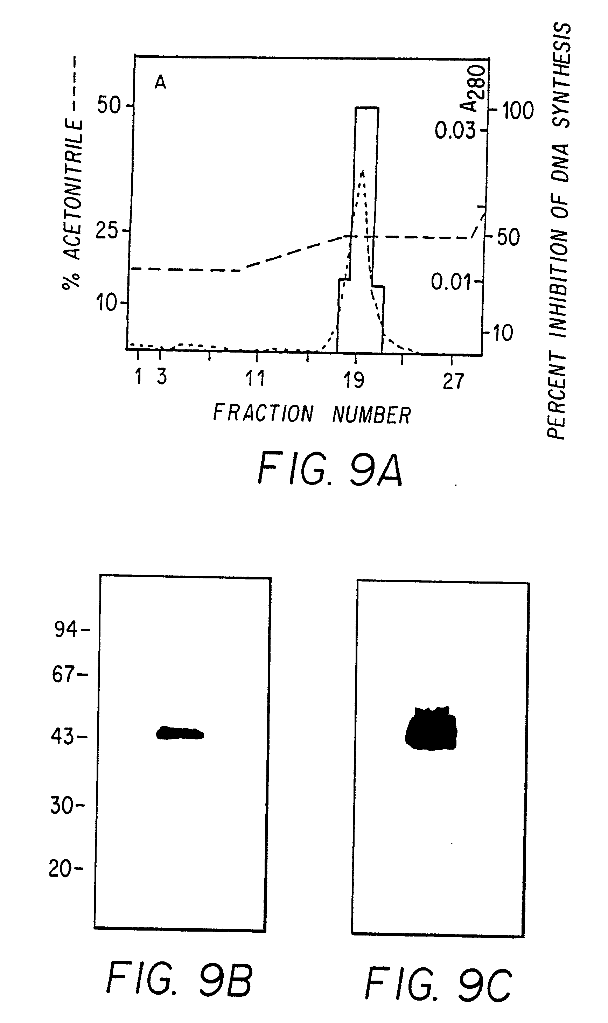

C/ Detection of IGF-I binding activity by IDF$_{45}$: About 300 ng of proteins are separated by SDS-PAGE and Western-blotted. The binding of the protein to $^{125}$I-IGF-I is detected by autoradiography as described in Figure 9C.

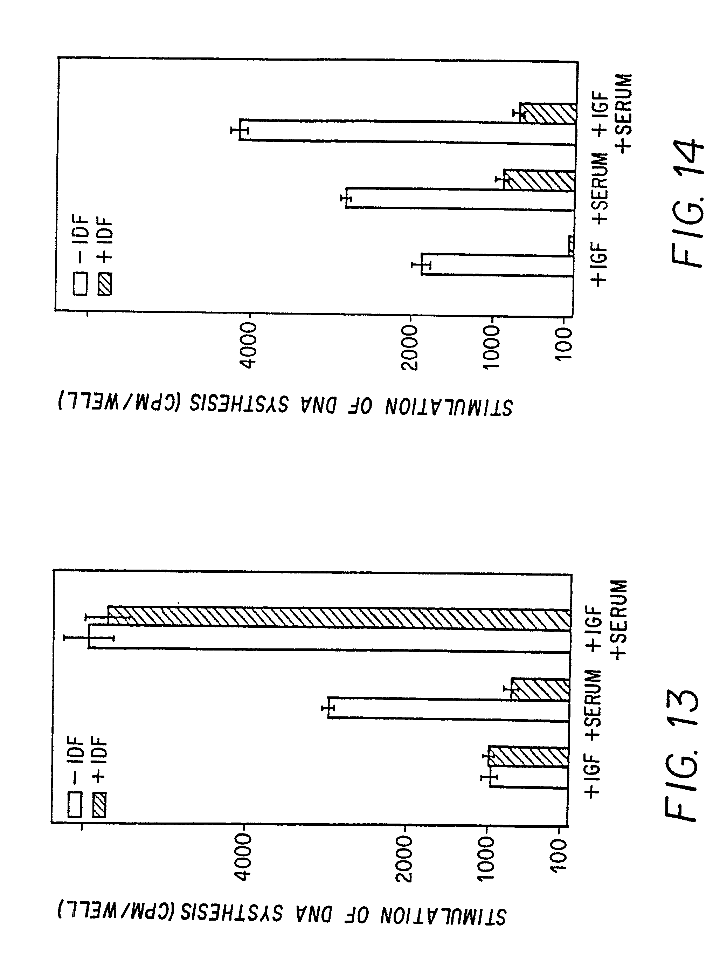

Figures 13 and 14 depict the inhibitory activity of IDF$_{45}$ in the presence of IGF-I as follows:

Figure 13 depicts the stimulation of DNA synthesis in CEF by IGF-I (80ng/ml) alone, serum (1%) alone or IGF-I (80ng/ml) plus serum (1%) in the absence or presence of IDF$_{45}$ (2U).

Figure 14 depicts the stimulation of DNA synthesis in CEF by IGF-I alone (40ng/ml instead of 80ng/ml), serum (1%) alone or IGF-I (40ng/ml) plus serum (1%) in the presence or absence of IDF$_{45}$ (2U).

## DETAILED DESCRIPTION OF THE INVENTION

The novel growth inhibitor of this invention is a protein with a molecular weight of 45 kD. The amino-terminal sequence of this protein, IDF$_{45}$, has no homology to known growth inhibitors.

The amino-terminal sequence of the first 12 amino acids of IDF$_{45}$ is as follows: Ser-Ala-Gly-Ala-Val-Gly-Ala-Gly-Pro-Val-Val-Arg.

The process for isolating and purifying the IDF$_{45}$ of this invention in substantially pure form from the source tissue comprises the sequence of steps of:

(a) preparation of conditioned medium from source tissue;

(b) concentration of the medium;

(c) gel filtration chromatography;

(d) a first reverse-phase HPLC; and

(e) a second reverse-phase HPLC.

Preparation of conditioned medium: In a preferred embodiment of this invention, the source tissue consists of Swiss 3T3 cells (American Type Culture Collection CCL92) and the conditioned medium is Dulbecco's modified Eagle Medium ("DMEM").

The use of other tissue sources to produce IDF$_{45}$ is within the scope of the invention using the techniques of those skilled in the art. It is also within the scope of the invention to obtain IDF$_{45}$ by other methods, including expression in transformed cells containing genetically engineered DNA vectors.

The Swiss 3T3 cells are grown at 37°C in DMEM containing 5% newborn calf serum and antibiotics (2). When the 3T3 cell culture becomes confluent, the medium is removed and replaced by serum free DMEM which is discarded 6 hours later. The cell culture is then washed with DMEM and kept at 37°C for 18 hours in the presence of serum-free DMEM. The culture is then slowly stirred for 15 minutes and the conditioned medium is removed and used as the source for the isolation of inhibitory molecules.

Concentration of macromolecules and separation of the medium: Three liters of conditioned medium are clarified by filtration on prefilter Millipore AP 20 and concentrated essentially as described by Holley et al. (3) by ultrafiltration on a YM10 Amicon filter (ultrafiltration cell model 2000). The upper surface of the membrane is rinsed first with 50 ml tris saline buffer (Tris-HCl 50 mM, NaCl 270 mM, KCl 10mM, Na$_2$HPO$_4$, 3.5 mM, pH 7.4) and then with 50 ml water in order to remove growth inhibitor attached to the membrane. The two eluants (50 ml) are combined and concentrated to 20 ml on a YM10 Amicon filter (ultrafiltration cell model 202). Finally, the upper surface of the large membrane is rinsed with 40 ml of 1M acetic acid and this eluant, combined with the last concentrate made to 1M with glacial acetic acid, is centrifuged (5000 rpm) for 15 minutes. The supernatant is concentrated to 5 ml on a YM10 Amicon filter.

Gel filtration: The macromolecules are fractionated by placing the concentrate on a P150 Bio-Gel chromatography column (20x5 cm) and eluted in 1M acetic acid at a flow rate of 21 ml/hour. Each fraction (8.5 ml) is lyophilized and an aliquot is tested for inhibitory activity. The results are shown in Figure 1A.

Reverse-Phase HPLC: The inhibitory fraction from P150 chromatography is dissolved in 1 ml of 0.05% TFA, adjusted to 17% acetonitrile and fractionated by two successive HPLC on a reverse-phase column C$_8$ Pro RPC HR 5/2 (Pharmacia).

In the first reverse-phase HPLC, elution is achieved with a multilinear gradient of acetonitrile in 0.05% TFA (17-24% acetonitrile for 9 minutes, 24% acetonitrile for 10 minutes and 24-100% acetonitrile for 15 minutes). Each fraction (0.3 ml) is lyophilized and an aliquot is tested for inhibitory activity. The results are shown in Figure 1B.

The inhibitory fraction from the first reverse-phase HPLC is adjusted to 17% acetonitrile in 0.05% TFA and rechromatographed in the same reverse- phase HPLC system. Elution is achieved with the same multilinear gradient of acetonitrile. Each fraction is lyophilized and an aliquot is tested for inhibitory activity. The results are shown in Figure IC.

Assay of IDF$_{45}$ activity: (A) Primary cultures of CEF are prepared from 10 day old brown Leghorn chick embryos and are cultivated in modified Eagle's medium and vitamins supplemented with antibiotics and 5% calf serum as described by Blat et al. (33). Secondary cultures of CEF are seeded at 8x10$^4$ cells/well in 96-well culture plates. Six hours later, medium is discarded and cells are maintained for 60 hours in serum-free medium. Fractions to be tested are solubilized in culture medium (alternatively in 0.5M acetic acid plus 200μg/ml albumin) and added on quiescent CEF culture in the presence of 1% serum. DNA synthesis is determined by labelling the cells with $^{14}$C-thymidine (0.07 μCi/well) between 5 and 24 hours after addition of the fraction to be tested and serum, as described (17). The results of the purification procedure are set forth in Table I:

Table 1 -

| IDF$_{45}$ Purification | | | | |
|---|---|---|---|---|
| Step of Purification | Protein (ug) | ED$_{50}$ ng/ml | Specific activity* U/ug | Total Activity recovered (U) |
| Soluble Protein after concentration from 3 liters CM** | 11,600 | $1 \times 10^5$ | 0.01 | 116 |
| Bio-GEL P150 chromatography*** | 1,200 | $2 \times 10^3$ | 0.5 | 600 |
| First FPLC reverse phase*** | 30 | 60 | 16.7 | 500 |
| Second FPLC reverse phase*** | 16 | 40 | 25.0 | 400 |

* One unit of activity (1U) is defined as the quantity of IDF$_{45}$ which inhibits 50% of DNA synthesis in CEF stimulated by 1% serum (ED$_{50}$).

** CM: Conditioned Medium

***These results are calculated taking account of the three most inhibitory fractions.

Table 1 summarizes the results of the different steps of purification. The acid-soluble proteins concentrated from conditioned medium are poorly inhibitory, and the 45% inhibition observed with 100 μg/ml protein does not increase when more protein is added. As shown previously (34), these proteins contain both inhibitory and stimulatory factors. After P150 Bio-Gel chromatography (Fig. 1A), a peak of inhibitory activity is found in fractions corresponding to a compound of about 45 kD, and recovery of inhibitory activity increases because it is separated from stimulatory activity. When the most inhibitory fractions of P150 Bio-Gel chromatography (Fig. 1A) are separated by reverse-phase HPLC, an inhibitory activity elutes with 24% acetonitrile (Fig. 1B and C). After the last step of purification, there is a 2,500-fold increase in specific activity compared to total acid-soluble proteins of conditioned medium.

(B) IDF$_{45}$ is also tested for activity on endothelial cells as follows: Bovine aortic endothelial cells are cultured as previously described (35,36,37). Cells are seeded in 24-well multiwell plates at a density of approximately 6,000 cells/well and grown for approximately 5 days in the presence of basic fibroblast growth factor ("bFGF") alone (4 ng/ml added on day one) or a combination of the same amount of bFGF and different amounts of IDF$_{45}$ (added on day one). At the end of the experiment, cells are trypsinized and counted using a Coulter particle counter.

A dose-response assay for the growth-inhibitory activity of IDF$_{45}$ on bFGF-stimulated endothelial cells is shown in Figure 2. It should be noted that IDF$_{45}$ is capable of overcoming the strong growth-stimulatory effect provided by a maximally active bFGF dose.

The inhibitory activity of IDF$_{45}$ on endothelial cells is also confirmed using a standard DNA synthesis assay (inhibition of radioactive thymidine incorporation into cellular DNA -- results not shown). Furthermore, IDF$_{45}$ also inhibits basal (serum-stimulated) DNA synthesis of endothelial cells in the absence of bFGF (results not shown).

Chemical and biological characterization of IDF$_{45}$: Lyophilized proteins are solubilized in a sodium dodecyl sulfate ("SDS") sample buffer (10% glycerol, 2.3% SDS, 0.0625 M Tris-HCl, pH 6.8) for SDS-PAGE or in NP40 0.1% for isoelectrofocusing. SDS-PAGE, isoelectrofocusing and silver staining are performed with a Phast System (Pharmacia) following the manufacturer's instructions. SDS-PAGE is performed by applying a sample of the protein to a gel slab together with a protein standard mixture containing phosphorylase b (94 kD), bovine serum albumin (67 kD), ovalbumin (46 kD), carbonic anhydrase (30 kD), soybean trypsin inhibitor (20 kD) and lactalbumin (14 kD).

When HPLC-purified bioactive material (Fig. 1C) is subjected to SDS-PAGE, a single band corresponding to a protein with a molecular weight of 45 kD is observed (Fig. 3). Under reducing conditions in the presence of dithiothreitol (0.065M), the activity of IDF$_{45}$ is destroyed (70% inhibition in the presence of non-treated IDF$_{45}$ v. 0% inhibition in the presence of treated IDF$_{45}$) and SDS-PAGE shows that the apparent molecular weight of the 45 kD protein shifts to 52 kd (results not shown). This behavior suggests that the conformation of the native monomer is constrained by intrachain disulfide linkages which are required for inhibitory activity. No glycoprotein is detected in purified IDF$_{45}$ when the method of Clegg (38) is used. Protein is determined from optical density at 280 nanometers with insulin used as a standard. Isoelectric focusing analysis shows that the pI of IDF$_{45}$ is about 6.5 (data not shown).

Amino terminal sequence: An Applied Biosystems Model 470A gas/liquid phase microsequenator is used to ascertain the amino-terminal sequence of IDF$_{45}$. Phenylthiohydantoin derivatives of amino acids are identified by reverse-phase HPLC on an Applied Biosystems Model 120A PTH amino-acid analyzer.

Procedures are carried out according to the manufacturer's experimental protocol.

The amino-terminal sequence of HPLC-purified IDF$_{45}$ is shown in Table 2 as follows:

Table 2:

| Amino-terminal Sequence of IDF$_{45}$ | | |
|---|---|---|
| Cycle # | <PhNCS* | pmol |
| 1 | Ser | 55 |
| 2 | Ala | 151 |
| 3 | Gly | 105 |
| 4 | Ala | 121 |
| 5 | Val | 87 |
| 6 | Gly | 131 |
| 7 | Ala | 111 |
| 8 | Gly | 72 |
| 9 | Pro | 66 |
| 10 | Val | 106 |
| 11 | Val | nd** |
| 12 | Arg | 38 |

* <PhNCS: Phenylthiohydantoin amino acid derivative.
** Quantitation of residue was not possible in this analysis.

Amino acid residues 1 - 12 are confirmed in an independent sequence analysis of another sample of IDF$_{45}$. Sequence analyses of a different IDF$_{45}$ preparation, using approximately 100-200 pmol of protein, also show the amino-terminal sequence to be Ser-Ala-Gly-Ala-Val-Gly-Ala-Gly-Pro-Val-Val-Arg.

Dose-response analysis: The inhibitory activity is determined by the percent inhibition of the increase of $^{14}$C-thymidine incorporation into CEF caused by 1% serum (Figure 4). In the absence of serum, $^{14}$C-thymidine incorporation is between 15 and 25% of the incorporation in the presence of serum. DNA synthesis is 100% inhibited in test cells when for $^{14}$C-thymidine incorporated in cells the readings are the same for control cells not stimulated by serum and for serum-stimulated test cells treated with IDF$_{45}$. The analysis indicates that 40 ng/ml of purified protein are necessary to decrease by 50% the stimulation of DNA synthesis induced in CEF by 1% serum. This corresponds to a concentration of about 1 nM IDF$_{45}$.

In view of these results, the role of IDF$_{45}$ in growth inhibition will now be discussed. The regulation of cell growth is probably under the control of positive and negative regulatory factors (1,39). IDF$_{45}$ may represent a negative regulator; it had been shown that partially-purified IDF$_{45}$ is not cytotoxic and its effect is reversible (18,19). Some evidence suggests that IDF$_{45}$ may function in an autocrine pathway. It had been shown for partially-purified IDF$_{45}$ (34) that dense cultures of 3T3 cells diffused, in the medium, both inhibitory and stimulatory factors which were separated by P150 Bio-Gel chromatography. The non-identified stimulatory activity which eluted with an apparent molecular weight of 10 kD was inhibited by molecules of 45 kd (34).

In order to explain the density-dependent inhibition of growth, it has been assumed (40) that, in the medium of dense quiescent cultures of 3T3 cells, inhibitory and stimulatory activities are in a balanced state. By contrast, in cultures of src-transformed cells, stimulatory factors are never counterbalanced by autocrine inhibitory molecules and cells go on to multiply until depletion of medium. In agreement with this hypothesis, it has been observed that cultures of cells transformed by v-src which have lost the density-dependent inhibition of growth (41) have also lost their sensitivity to IDF$_{45}$ (19).

In another aspect of this invention, applicants show that IDF$_{45}$ is, after Western blotting, able to bind IGF-I. Furthermore, in CEF, DNA synthesis stimulated by IGF-I is 100% inhibited in the presence of purified IDF$_{45}$.

The analysis of the binding of IDF$_{45}$ to IGF-I to cause inhibition, either alone, or in comparison with inhibition of serum stimulation, is carried out in the following series of experiments:

(a) Stimulation of growth of CEF by addition of IGF-I or serum in the absence of IGF-I. This serves as a baseline for later experiments.

(b) Inhibition of IGF-I stimulation by IDF$_{45}$ at different concentrations and temperatures.

(c) Comparison of inhibition of IGF-I and serum stimulation by IDF$_{45}$ at different concentrations of IGF-I alone or in the presence of serum.

Stimulation by IGF-I and serum of DNA synthesis in CEF: Different concentrations of IGF-I are added to quiescent CEF, and DNA synthesis is determined by labelling the cells between 5 and 24 hours after addition of IGF-I (Figure 6). IGF-I was purchased from Amersham. Stimulation of DNA synthesis reaches a plateau at a concentration of 20 ng/ml IGF-I. At this concentration, DNA synthesis is 130% stimulated.

In contrast, using the same system, stimulation by serum is more pronounced. Figure 6 shows that 0.8% serum induces greater stimulation (320%) of DNA synthesis than that induced by IGF-I. These results establish a baseline for the results set forth below.

Inhibition by IDF$_{45}$ of IGF-I stimulation: DNA synthesis is determined in CEF stimulated by 30 ng/ml IGF-I in the presence of different concentrations of purified IDF$_{45}$ added at time 0 with IGF-I. In this experiment, DNA synthesis is 140% stimulated by IGF-I. The inhibition induced by IDF$_{45}$ is proportional to the IDF$_{45}$ concentration and reaches 100% at a concentration of 2 U IDF$_{45}$ (Figure 7). At this concentration, IDF$_{45}$ inhibits the stimulation induced by 1% serum by only 66% (results not shown).

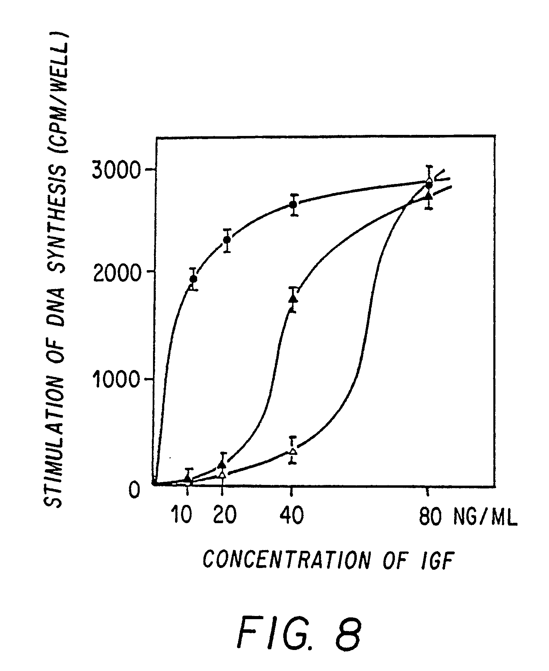

In the experiment shown in Figure 8, 1 unit or 1.5 units of IDF$_{45}$ are added to cells in the presence of different concentrations of IGF-I. Stimulation of DNA synthesis induced by IGF-I is 95% inhibited when 10ng/ml of IGF-I are added, but inhibition decreases when the concentration of IGF-I increases and no inhibition is observed when 80 ng/ml IGF-I are added to the medium. The data from Figures 7 and 8 suggest that inhibition by IDF$_{45}$ of the stimulation induced by IGF-I is the result of binding by IDF$_{45}$ of IGF-I. The results set forth below verify this assumption.

IGF binding property of purified IDF$_{45}$: After the final step in IDF$_{45}$ purification by reverse-phase FPLC described previously, each fraction is tested for the inhibitory activity of the DNA synthesis stimulated by 1% serum (Figure 9A). Lyophilized proteins are subjected to SDS-PAGE using the procedure described previously.

As observed in Figure 9B, after SDS-PAGE of fractions containing IDF$_{45}$, one band of 45 Kd protein is silver-stained. The protein is then subjected to Western blotting as follows: After SDS-PAGE, protein is transferred by passive diffusion on a nitrocellulose sheet. The gel plus one sheet of nitrocellulose (0.2 um Schleicher and Schuell BA 83) and two sheets of paper (Whatman 3MM) are soaked for 5 minutes in PBS buffer. On a glass slab are set successively the gel, the wet nitrocellulose sheet, the two wet sheets of paper, two dried sheets of paper (Whatman 3MM) and a glass slab. The apparatus is compressed by pressure of 40 g/cm². After 48 hours at room temperature, about 80% of the total protein applied to the gel is recovered on the nitrocellulose membrane. Following this, nitrocellulose is dried at 37°C for 15 minutes and processed essentially as described by Hossenlopp et al. (32) to detect IGF binding activity. After Western blotting, this protein binds $^{125}$I-IGF-I purchased from Amersham (Figure 9C). Based on this observation, it appears that inhibition by IDF$_{45}$ of IGF-I stimulated DNA synthesis is the result of its property to bind IGF-I.

Since IGF-I is a component of serum, it is thus of interest to determine, whether, in CEF, inhibition by IDF of DNA synthesis stimulated by 1% serum is the result of the inhibitory activity of IDF$_{45}$ upon IGF-I stimulation. The results shown in Figures 10-11 indicate that this is not the case.

Additive effect of serum and IGF-I on DNA synthesis in the absence of IDF$_{45}$: DNA synthesis in CEF is determined in the presence of 40 or 80 ng/ml IGF-I with and without 1% serum, in the absence of IDF$_{45}$ - (Figure 10) according to the method of Figure 7.

DNA synthesis is 200% stimulated at 40 and 80 ng IGF-I/ml. It is 400% stimulated by 1% calf serum alone and 600% stimulated by serum plus IGF-I. Stimulation of DNA synthesis by serum and IGF-I is thus additive, indicating that stimulation by serum is not simply the result of stimulation by the IGF-I present in the serum. Further support is provided by the results from Figure 11, which are now discussed.

Effect of IDF$_{45}$ on stimulation induced by serum and IGF-I in transformed CEF: CEF infected by NY68 virus (a mutant of Rous sarcoma virus thermosensitive for the expression of the oncogene v-src) (42) are concentrated to 5X10$^4$ cells/well and are seeded at 37°C in Eagle's medium plus 4% serum. The cells are then transferred to 41°C 24 hours later and maintained at this temperature for 3 days. The medium is replaced by serum free medium (100μl) 18 hours before the experiment and cells maintained at 41°C. Then, one part of the culture is transferred to 37°C; 4 hours later, IDF$_{45}$ with or without 1% serum is added to the cultures either maintained at 41°C or transferred to 37°C. DNA synthesis is determined by labelling the cells between 5 and 24 hours after addition of IDF$_{45}$. Data in each experiment are the mean of 3 determinations (±SD).

In the system shown in Figure 11A, cultures are either maintained at the restrictive temperature (41°C) or transferred to the permissive temperature (37°) for the expression of the v-src oncogene. Stimulation of DNA synthesis by IGF-I and serum is determined in the presence or absence of IDF$_{45}$. The expression of v-src in CEF infected by NY68 virus and transferred from 41 to 37°C in the absence of serum stimulates DNA synthesis by 300% but this stimulation is not inhibited by IDF$_{45}$ (Figure 11A).

DNA synthesis is stimulated by IGF-I or by serum in cells maintained at 41°C or transferred to 37°C. In cells maintained at 41°C, IGF-I and serum stimulation of DNA synthesis are each inhibited by IDF$_{45}$ - (Figure 11b). In cells transferred to the permissive temperature for the expression of v-src, IGF-I stimulation is 100% inhibited, whereas stimulation induced by serum is not significantly inhibited (Figure 11C), as previously observed.

Taken together, these data indicate that purified IDF$_{45}$ has two functions: to inhibit IGF-I stimulation and to inhibit serum stimulation.

Purification of IDF$_{45}$ by cation exchange FPLC: IDF$_{45}$ is purified as previously described, except that the final step of purification by reverse-phase FPLC is replaced by purification on a cation exchange column (Mono S HR5/5, Pharmacia), using conditions set forth in the detailed description of Figure 12. Each fraction is tested for the inhibitory activity of DNA synthesis stimulated by 1% serum (Figure 12A). Proteins of the most inhibitory fraction are separated by SDS-PAGE and silver-stained or Western blotted.

The major 45 kD protein (IDF$_{45}$) is silver stained (Figure 12B). This protein has the property of binding $^{125}$I-IGF-I, as revealed by autoradiography (Figure 12C). Therefore, IDF$_{45}$, which has the capacity to bind IGF-I and to inhibit DNA synthesis stimulated by serum, is obtained whatever the method of purification used.

Inhibitory activity of IDF$_{45}$ upon serum in the presence of high concentrations of IGF-I: Inhibition by IDF of IGF-I stimulation of DNA synthesis is the result of the IGF-I binding activity of IDF$_{45}$. As shown in Figure 8, in the presence of 80 ng/ml of IGF-I, most of the IDF$_{45}$ molecules (likely bound to IGF-I) are unable to inhibit stimulation induced by the free IGF-I molecules. The results of Figures 13 and 14 indicate that whether IDF$_{45}$ bound to IGF-I is able to inhibit serum stimulation depends upon the concentration of IGF-I.

In Figure 13, CEF is stimulated by serum (1%) alone, IGF-I (80 ng/ml) alone or by the mixture of IGF-I (80 ng/ml) and serum (1%) in the presence or absence of IDF$_{45}$ (2U). DNA synthesis is 100% stimulated by IGF-I alone, 260% by serum alone and 600% by IGF-I plus serum. IDF$_{45}$ serves to inhibit the stimulation of DNA synthesis only for serum alone.

Stimulation of DNA synthesis by IGF-I alone is not inhibited by IDF$_{45}$. This result agrees with that shown in Figure 8. DNA synthesis stimulated by serum alone is 73% inhibited; however, when serum and IGF-I are added simultaneously, DNA synthesis is not significantly inhibited (Fig 13). The latter result clearly shows that IDF$_{45}$ is unable to inhibit serum stimulated DNA synthesis in the presence of a large concentration of IGF-I.

In Figure 14, CEF are stimulated by IGF-I alone (40 ng/ml instead of 80 ng/ml), serum (1%) alone or IGF-I (40 ng/ml) plus serum (1%) in the presence or absence of IDF$_{45}$ (2U). DNA synthesis is 150% stimulated by IGF-I alone, 220% by serum alone and 330% by serum plus IGF-I. IDF$_{45}$ serves to inhibit the stimulation of DNA synthesis in all cases due to the reduced concentration of IGF-I. Therefore, the inhibitory activity of IDF$_{45}$ upon serum stimulation depends upon the IGF-I concentration present in medium.

The foregoing results demonstrate that, in CEF, the stimulation of DNA synthesis by IGF-I is 100% inhibited in the presence of IDF$_{45}$. Furthermore, purified IDF$_{45}$, which after SDS-PAGE shows only one protein band of 45 kD, is able to bind IGF-I.

The inhibitory effect of IDF$_{45}$ upon serum stimulation does not seem to result from its inhibitory effect upon IGF-I stimulation. Stimulation of DNA synthesis by IGF-I and by serum is additive; indeed, in some experiments, a synergy between the two effects is observed. This suggests that the mode of action of IGF-I and serum are different, and that stimulation by serum is not due to cell stimulation by IGF-I present in the serum. Moreover, it is possible to dissociate the two inhibitory effects of IDF$_{45}$ on IGF-I and serum stimulation. IDF$_{45}$ inhibits the stimulation induced by serum or IGF-I when added to NY68 infected CEF, maintained at the restrictive temperature for the expression of v-src, whereas IDF$_{45}$, when added to v-scr transformed CEF, is able to inhibit stimulation induced by IGF-I but is unable to significantly decrease serum induced stimulation. These results agree with those reported previously (19) and also with the assumption that inhibition by IDF of IGF-I stimulation is due to binding of IGF-I to IDF$_{45}$. Indeed, in this case, IDF$_{45}$ would bind IGF-I in the culture medium and would prevent IGF-I stimulation whatever the cell type present in the culture.

Taken together, these results indicate that IDF$_{45}$ has two different functions, one of which is to inhibit serum stimulation, and the other to bind IGF-I. This conclusion is supported by two additional results.

First, it is impossible to separate the two activities when is IDF$_{45}$ is purified by cation exchange FPLC rather than reverse-phase FPLC. Second, IDF$_{45}$ when bound to IGF-I (in the presence of a large concentration of IGF-I), is unable to inhibit stimulation of DNA synthesis induced by serum. Indeed, as shown in Figures 13 and 14, the inhibitory activity of IDF$_{45}$ upon IGF-I stimulation depends upon the IGF concentration. One unit of IDF$_{45}$ (about 1nM) mainly inhibits stimulation induced by 2 nM IGF-I. When the concentration of IGF-I increases and is higher than two nM, inhibition decreases because IDF$_{45}$ bound to IGF-I is unable to inhibit added free IGF-I.

If the inhibitory effect upon serum stimulation and IGF binding activity is carried on two different proteins, the presence of IGF-I, under conditions in which most of the IDF$_{45}$ is bound to IGF-I, should have no effect upon the activity of the molecule which inhibits stimulation induced by serum. In fact, the contrary is observed: when IDF$_{45}$ binds to IGF-I, it loses its inhibitory effect on stimulation induced by serum. These results thus strongly indicate that the two activities take place on the same protein.

In conclusion, IDF$_{45}$ is a bifunctional protein able to inhibit serum stimulation and to bind IGF-I.

It should be noted that other bifunctional proteins are known (43). One example is TGF-B, which has both inhibitory and stimulatory activities (3,4,5,6). Another is the IGF-II receptor, which has the property not only of binding IGF-II but also of binding and transporting mannose-6 phosphate (44,45). However, none of these proteins binds to IGF-I.

IDF$_{45}$ is clearly distinguishable from other known growth inhibitors on the basis of molecular weight, amino-terminal sequence and, in some instances, cell specificity. For example, IDF$_{45}$ differs from TGF-$\beta$ by molecular weight, amino-terminal sequence and action on fibroblasts. TGF-$\beta$ is inhibitory for epithelial cells (3) but, unlike IDF$_{45}$, is stimulatory for CEF (42) and 3T3 cells (46). The amino-terminal sequence of IDF$_{45}$ differs from that of the family of TGF-$\beta$ proteins (6).

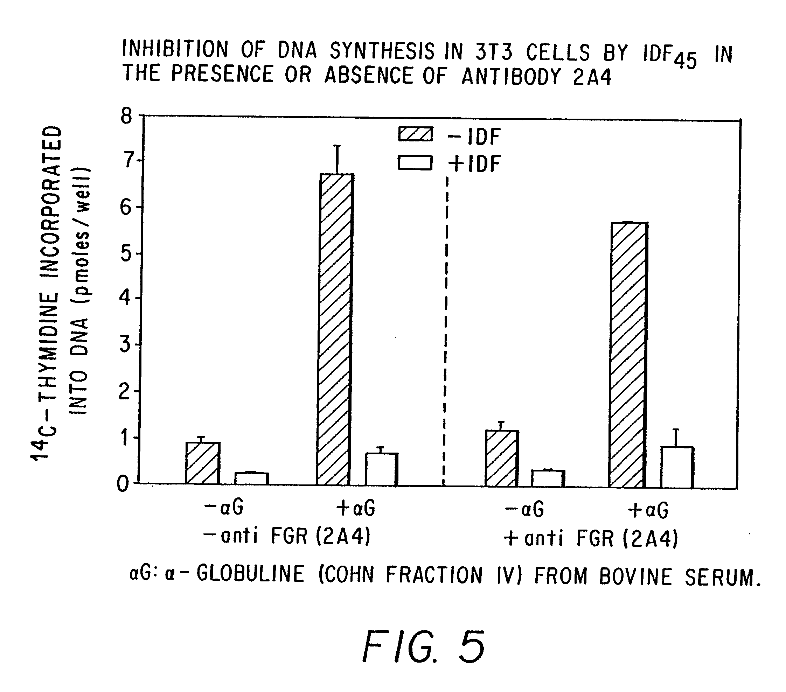

IDF$_{45}$ also differs from the 13K FGR as follows: (a) a monoclonal antibody which specifically binds and neutralizes the 13K FGR (15) is unable to neutralize the inhibitory activity of IDF$_{45}$ (Figure 5); and (b) the amino-terminal sequences and molecular weights of the two substances are clearly distinguishable (14). IDF$_{45}$ also differs in a similar manner from mammary-derived growth inhibitor (14), which shares a common structural feature with FGR (16).

Similarly, IDF$_{45}$ is also distinguishable from other growth inhibitors such as interferons, interleukin-1 and tissue necrosis factor-$\alpha$ on the basis of differences in molecular weight, amino-terminal sequence and, in some instances, cell specificity of inhibitory activity (47,48).

Therapeutic compositions in accordance with this invention include IDF$_{45}$ dispersed in a conventional pharmaceutically acceptable liquid or solid carrier. The therapeutic compositions may be administered topically in the form of creams, lotions and so forth, or orally in such forms as tablets, capsules, dispersible powders, granules or suspensions, or parenterally in the form of sterile injectable solutions or suspensions. These therapeutic compositions may be administered to human or veterinary patients to inhibit growth of cells. The compositions may also be applied to tissues removed from the patient for treatment; said tissues may then be reinserted into the patient.

## REFERENCES

(1) Harel, L., Growth Factors Handbook of Exp. Pharmacology, 57, 313-341, Ed. R. Baserga (1981) (Springer-Verlag, Heidelberg).

(2) Harel, L., et al., J. Cell. Physiol., 119, 101-106 (1984).

(3) Holley, R.W., et al., Proc. Nat. Acad. Sci., 77, 5989-5992 (1980).

(4) Tucker, R.F., et al., Science, 226, 705-707 (1984).

(5) Roberts, A.B., et al., Proc. Nat. Acad. Sci., 82, 119-123 (1985).

(6) Hanks, S.K., et al., Proc. Nat. Acad. Sci., 85, 79-82 (1988).

(7) Gresser, I. and Tovey, M. G., Biochem. & Biophys. Acta, 516, 231-247 (1978).

(8) Niitsu, Y., et al., Japan J. Cancer Res., 76, 1193-1197 (1985).

(9) Sugarman, B.J., et al., Science, 230, 943-945 (1985).

(10) Stolpen, A. H., et al., Am. J. Pathol., 123, 16-24 (1986).

(11) Sato, N., et al., J. Nat. Cancer Inst., 76, 1113-1121 (1986).

(12) Tsai, S.C. and Gaffney, E.V., Cancer Research, 46, 1471-1477 (1986).

(13) Hsu, Y.M. and Wang, J.L., J. Cell. Biol., 102, 362-368 (1986).

(14) Bohmer, F.D., et al., J. Biol. Chem., 262, 15137-15143 (1987).

(15) Hsu, Y.M., et al., Proc. Nat. Acad. Sci., 81, 2107-2111 (1984).

(16) Bohmer, F.D., et al., Biochem. Biophys. Research Comm., 148, 1425-1431 (1987).

(17) Harel, L., et al., Biol. of the Cell, 48, 11 (1983).

(18) Blat, C., et al., FEBS Lett., 203-2, 175-180 (1986).

(19) Blat, C., et al., J. Cell. Physiol., 130, 416-419 (1987).

(20) Baxter, R.C. and Martin, J.L., Prog. in Growth Factor Res., 1, 49-68 (1989).

(21) Zapf, J., et al., Curr. Top. Cell. Regul., 19, 257-309 (1981).

(22) Moses, A.C., et al., Nature, 263, 137-140 (1976).

(23) Nissley, S.P. and Rechler, M.M., Hormonal Proteins and Peptides, 12, 127-203 (1984).

(24) Hardouin, S., et al., Eur. J. Biochem., 170, 121-132 (1987).

(25) Lyons, R.M. and Smith, G.L. Mol. Cell. Endocrinol., 45, 263-270 (1986).

(26) Mottola, C., et al., J. Biol. Chem., 261, 11180-11188 (1986).

(27) Romanus, J.A., et al., Endocronology, 121, 1041-1050 (1987).

(28) Hossenlopp, P., et al., Eur. J. Biochem., 170, 133-142 (1987).

(29) Clemmons, D.R., et al., J. Clin. Invest., 77, 1548-1553 (1986).

(30) Brinkman, A., et al., Embo. J., 7, 2417-2423 (1988).

(31) Brewer, M.T., et al., Biochem. Biophys. Res. Comm., 152, 1289-1297 (1988).

(32) Hossenlopp, P., et al., Ana. Biochem., 154, 138-143 (1986).

(33) Blat, C., et al., Exp. Cell. Res., 134, 121 (1981).

(34) Harel, L., et al., J. Cell. Physiol., 123, 139-143 (1985).

(35) Gospodarowicz, D., et al., Proc. Nat. Acad. Sci., 81, 6963-6967 (1984).

(36) Bohlen, P., et al., EMBO J., 4, 1951-1956 (1985).

(37) Bohlen, P., et al., Proc. Nat. Acad. Sci., 81, 5364-5368 (1984).

(38) Clegg, J.C.S., Analyt. Biochem., 127, 389-394 (1982).

(39) Sporn, M.B., and Roberts, A., Nature, 313, 745-747 (1985).

(40) Blat, C., et al., CR Acad. Science (Paris), 301 III 9, 417-422 (1985).

(41) Jullien, M., et al., Exp. Cell Research, 152, 390-401 (1984).

(42) Blat, C., et al., Cell. Biol. Inter. Reports, 10, 947-954 (1986).

(43) Sporn, M. and Roberts, A., Nature, 332, 217-219 (1988).

(44) MacDonald, R.G., et al., Science, 239, 1134-1137 (1988).

(45) Morgan, D.O., et al., Nature, 329, 301-307 (1987).

(46) Brown, K. D., and Holley, R. N., Proc. Nat. Acad. Sci., 84, 3743-7 (1987).

(47) Pennica, D., Nature, 312, 724-729 (1985).

(48) Frater-Schroeder, M. et al., Proc. Nat. Acad. Sci., 84, 5277-5281 (1987).

**Claims**

1. An inhibitory diffusible factor in substantially pure form having the amino-terminal sequence H-Ser-Ala-Gly-Ala-Val-Gly-Ala-Gly-Pro-Val-Val-Arg-.

2. An inhibitory diffusible factor according to claim 1 which has a molecular weight of about 45,000 daltons.

3. An inhibitory diffusible factor according to claim 2 which is isolated from mouse 3T3 cells.

4. A method for the preparation and isolation of an inhibitory diffusible factor in substantially pure form which comprises the sequence of steps of:

(a) preparing a conditioned medium from the source tissue;

(b) concentrating said medium;

(c) gel filtration chromatography;

(d) a first reverse-phase high performance liquid chromatography of fractions of step (c); and

(e) a second reverse-phase high performance liquid chromatography of fractions of step (d).

5. The method of claim 4 wherein the eluted fractions of steps (c), (d) and (e) are analyzed for the presence of an inhibitory diffusible factor by testing each fraction for inhibitory activity of DNA synthesis in chick embryo fibroblasts.

6. The method of claim 5 wherein the source tissue is mouse 3T3 cells.

7. A therapeutic composition of matter useful for inhibiting growth in fibroblasts which comprises a pharmaceutical carrier and an effective fibroblast growth inhibiting amount of the inhibitory diffusible factor of claim 1.

8. A therapeutic composition of matter useful for inhibiting growth in endothelial cells which comprises a pharmaceutical carrier and an effective endothelial cell growth inhibiting amount of the inhibitory diffusible factor of claim 1.

9. A therapeutic composition of matter useful for inhibiting DNA synthesis which comprises a pharmaceutical carrier and an effective DNA synthesis inhibiting amount of the inhibitory diffusible factor of claim 1.

10. A thereapeutic composition of matter useful for inhibiting the activity of insulin-like growth factor I which comprises a pharmaceutical carrier and the inhibitory diffusible factor of claim 1 in an amount effective to bind to and thereby inhibit the activity of insulin-like growth factor I.

FIG. 1A

FIG. 1B

FIG. 1C

INHIBITION OF ENDOTHELIAL CELL GROWTH BY IDF-45

*FIG. 2*

*FIG. 3*

FIG. 4

INHIBITION OF DNA SYNTHESIS IN 3T3 CELLS BY $IDF_{45}$ IN THE PRESENCE OR ABSENCE OF ANTIBODY 2A4

αG: α-GLOBULINE (COHN FRACTION IV) FROM BOVINE SERUM.

*FIG. 5*

EP 0 391 214 A1

FIG. 6

FIG. 7

FIG. 8

FIG. 9A

FIG. 9B

FIG. 9C

FIG. 10

FIG. 11A

FIG. 11B

FIG. 11C

FIG. 12A

FIG. 12B

FIG. 12C

FIG. 13

FIG. 14

EP 0 391 214 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | No relevant documents have been disclosed. | | C 07 K 15/12 <br> A 61 K 37/02 |
| | | | **TECHNICAL FIELDS SEARCHED (Int. Cl.5)** |
| | | | C 07 K <br> A 61 K |

The present search report has been drawn up for all claims

| Place of search | Date of completion of the search | Examiner |
|---|---|---|
| THE HAGUE | 04-07-1990 | RAJIC M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.................................................................................
& : member of the same patent family, corresponding
   document

EPO FORM 1503 03.82 (P0401)